# NOUVEAU FASCICULE DE BREVET EUROPEEN

(11) **EP 0 302 769 B2**
(45) Date de publication et mention de la décision concernant l'opposition: **23.08.2006**
(45) Mention de la délivrance du brevet: 10.07.1991
(21) Numéro de dépôt: 88401810.2
(22) Date de dépôt: 12.07.1988
(51) Int. Cl.: A61K 31/23, A23L 1/30, A23D 7/00

(54) **Emulsion lipidique destinée à la nutrition parentérale ou entérale**
Für die parenterale oder enterale Ernährung bestimmte Lipidemulsion
Lipid emulsion for parenteral or enteral nutrition

(30) Priorité: 23.07.1987 FR 8710407
(43) Date de publication de la demande: 08.02.1989
(73) Titulaire: CLINTEC NUTRITION COMPANY, Deerfield, Illinois 60015 (US)
(72) Inventeur: Dutot, Guy, F-78590 Noisy le Roi (FR)
(74) Mandataire: Dossmann, Gérard

(56) Documents cités:
- EP-A- 0 189 160
- EP-A- 0 216 419
- WO-A-86/01715
- DE-A- 3 230 103
- FR-A- 2 097 036
- DIALOG 76135111 EMBASE; L. VALENZANO et al.: "The use of avocado and soya extracts in dermatological therapy" & G. ITAL. DERM. MINERVA DERM (ITALY), 1975, 110/5 (212-214)

## Description

La présente invention a pour objet une émulsion lipidique destinée à la nutrition parentérale ou entérale.

La justification de l'emploi des lipides en nutrition parentérale repose sur les arguments suivants:
- un apport calorique mixte glucidique-lipidique est plus physiologique qu'un apport glucidique exclusif,
- les lipides fournissent les acides gras essentiels que l'organisme est incapable de synthétiser.
- un fort pouvoir calorique associé à une faible osmolarité permet d'administrer une grande quantité de calories sous un faible volume par voie périphérique.

Les huiles de soja et de carthame actuellement utilisées dans les émulsions lipidiques injectables sont charactérisées par une forte teneur en acides gras polyinsaturés (environ 60% et 75% respectivement) qui permettent de couvrir largement les besoins en acides gras essentiels du patient nécessitant une nutrition intraveineuse.

Cependant, dans le cadre d'une nutrition parentérale totale où l'apport lipidique représente 30 à 50% des calories glucido-lipidiques, la composition de ces huiles est inadaptée pour les raisons suivantes :
- la forte proportion d'acides gras essentiels semble susceptible d'inhiber partiellement leur désaturation/élongation vers les métabolites supérieurs actifs (S. INNIS, Lipids, 1986, 21, 132-138).
- la forte teneur en acide linoléique de ces huiles a été rendue responsable d'effets immuno-suppresseurs qui peuvent interagir avec les phénomènes inflammatoires préexistant chez le patient en situation de stress.
- la composition en acides gras des huiles de carthame et de soja les rend très sensibles à la peroxydation. Les acides linoléique et α-linolénique sont très sensibles à la peroxydation en raison de la présence de plusieurs doubles liaisons dans leur structure. La peroxydation des acides gras engendra la formation de composés divers mal métabolisés par l'organisme.

Certains dérivés d'oxydation des acides gras réagissent avec les protéines pour donner naissance à des produits de condensation fluorescents appelés lipofuscines qui se déposent dans les tissus. De tels pigments ont été décrits sous nutrition parentérale.

Selon certains auteurs, ils pourraient être responsables de certaines anomalies hépatiques observées chez les patients recevant une nutrition parentérale.

L'augmentation de la peroxydation lipidique, lors de l'administration d'émulsions lipidiques, a été démontrée tant chez l'homme que chez l'animal (J.R. WISPE, Ped., 1985, 19, 374-379). Ces phénomènes de peroxydation risquent d'être amplifiés chez le sujet en situation de stress où la production de radicaux libres est augmentée avec parfois, une diminution d'activité des mécanismes de protection antioxydants (M. HIRA-MATSU, Bums, 1984, 11, 111-116).

Le document WO-A-66 01 715 a pour objet des compositions pharmaceutiques destinées à l'administration entérale au parentérale et comprenant un lipide structuré constitué de triglycérides dont les acides ont des chaînes C₈-C₁₂ et C₁₂-C₁₈.

Le document DE-A-3 230103 décrit des compositions diététiques contenant des mélanges d'huiles et dont la teneur en acides gras essentiels est > 50%.

La présente invention a pour objet une émulsion lipidique plus équilibrée à teneur réduite en acides gras polyinsaturés qui, tout en assurant la couverture des besoins en acides gras essentiels, présente les avantages suivants :
- meilleure utilisation des acides gras essentiels vers leurs dérivés supérieurs en évitant le risque d'inhibition de conversion des acides gras par excès de substrat,
- apport moins important d'acides gras polyinsaturés permettant de limiter la peroxydation des lipides, notamment chez des sujets atteints de syndromes inflammatoires, avec production de radicaux libres. L'émulsion lipidique de l'invention se caractérise par le fait que la phase lipidique obtenue par mélange de deux ou plusieurs des huiles suivantes : abricot, amande, arachide, avocat, blé, carthame, colza, coton, lupin, maïs, noisette, noix, olive, onagre palme, pêche, raisin, riz, seigle, sésame, soja, tournesol, tomate, lin, citrus, est un mélange d'acides gras à chaîne longue (C > 12) dont 15 à 45 % du total des acides gras sont des acides gras essentiels et qu'elle contient comme agents émulsifiants des phospholipides végétaux, animaux ou de synthèse.

| EMULSION LIPIDIQUE (MELANGE D'HUILES) | acides gras essentiels en % des acides gras totaux |
|---|---|
| AVOCAT (46 %) - SOJA (54 %) | 40 |
| OLIVE (47 %) - SOJA (53 %) | 40 |
| AVOCAT (60 %) - NOIX (40 %) | 40 |
| AVOCAT (89 %) - NOIX (11 %) | 20 |
| NOIX (11 %) - OLIVE (89 %) | 20 |
| OLIVE (85 %) - SOJA (15 %) | 20 |
| NOIX (40 %) - OLIVE (60 %) | 40 |

Des variations dans la composition des huiles peuvent être observées en fonction de leur prevenance. Les langes seront donc adaptés pour obtenir la composition en acides gras essentiels requise.

Les émulsions lipidiques de l'invention sont des émulsions contenant de 5 à 50% en poids d'huiles dans de l'eau. Les agents émulsifiants contenus par les émulsions de l'invention sont, par exemple, des phospholipides végétaux ou animaux ou de synthèse et sont contenus à raison de 0.5 à 5% en poids de l'émulsion.

Les émulsions de l'invention contiennent également un agent isotonisant tel que glycérol, glucose, un polyol, des acides aminés.

Les émulsions lipidiques de l'invention peuvent être utilisées en nutrition entérale ou parentérale en tant que telles ou en tant que constituant lipidique d'une émulsion ou solution complète contenant, par ailleurs, des acides aminés, des glucides, des vitamines, de la carnitine, des oligoéléments, des cétoanalogues d'acides aminés.

Les émulsions ou solutions complètes, contenant parmi leurs constituants les émulsions lipidiques de l'invention, font également partie de l'invention.

Les émulsions lipidiques de l'invention peuvent être utilisées pour réaliser un apport de calories et d'acides gras essentiels par voie parentérale ou entérale lorsqu'une alimentation orale est impossible.

## Revendications

1. Emulsion lipidique destinée à la nutrition parentérale ou entérale, **caractérisée par le fait que** la phase lipidique, obtenue par mélange de deux ou plusieurs des huiles suivantes : abricot, amande, arachide, avocat, blé, carthame, colza, coton, lupin, maïs, noisette, noix, olive, onagre, palme, pêche, raisin, riz, seigle, sésame, soja, tournesol, tomate, lin, citrus est un mélange d'acides gras à chaîne longue (C > 12) dont 15 à 45 % du total des acides gras sont des acides gras essentiels et qu'elle contient comme agents émulsifiants des phospholipides végétaux, animaux ou de synthèse.

2. Emulsion selon la revendication 1 ou la revendication 2, **caractérisée par le fait qu'**elle contient un mélange d'huiles d'avocat (46%) et de soja (54%).

3. Emulsion lipidique selon la revendication 1 ou 2, **caractérisée par le fait qu'**elle contient un mélange d'huiles d'olive (47%) et de soja (53%).

4. Emulsion lipidique selon la revendication 1 ou 2, **caractérisée par le fait qu'**elle contient un mélange d'huiles d'avocat (60%) et de noix (40%).

5. Emulsion lipidique selon la revendication 1 ou 2, **caractérisée par le fait qu'**elle contient un mélange d'huiles d'avocat (89%) et de noix (11%).

6. Emulsion lipidique selon la revendication 1 ou 2, **caractérisée par le fait qu'**elle contient un mélange d'huiles de noix (11%) et d'olive (89%).

7. Emulsion lipidique selon la revendication 1 ou 2, **caractérisée par le fait qu'**elle contient un mélange d'huiles d'olive (85%) et de soja (15%).

8. Emulsion lipidique selon la revendication 1 ou 2, **caractérisée par le fait qu'**elle contient un mélange d'huiles de noix (40%) et d'olive (60%).

9. Emulsion lipidique selon l'une quelconque des revendications 1 à 8, **caractérisée par le fait qu'**elle contient de 5 à 50% en poids d'huiles dans l'eau.

10. Emulsion lipidique selon l'une quelconque des revendications 1 à 9, **caractérisée par le fait qu'**elle contient comme agents émulsifiants des phospholipides végétaux ou aninaux ou de synthèse, à raison de 0,5 à 5% en poids de l'émulsion.

11. Emulsion lipidique selon l'une quelconque des revendications 1 à 10 **caractérisée par le fait qu'**elle contient un agent isotonisant.

12. Emulsion ou solution destinée à l'alimentation entérale ou parentérale, **caractérisée par le fait qu'**elle contient, parmi ses constituants, l'émulsion lipidique définie dans l'une quelconque des revendications 1 à 12.

13. Composition pharmaceutique **caractérisée en ce qu'**elle est constituée par une émulsion lipidique telle que définie dans l'une quelconque des revendications 1 à 11.

14. Composition pharmaceutique **caractérisée en ce qu'**elle contient, à titre de constituant, une émulsion lipidique telle que définie dans l'une quelconque des revendications 1 à 11.

15. Composition diététique **caractérisée en ce qu'**elle est constituée par une émulsion lipidique telle que définis dans l'une quelconque des revendications 1 à 11.

16. Composition diététique **caractérisée en ce qu'**elle contient, à titre de constituant, une émulsion lipidique telle que définie dans l'une quelconque des revendications 1 à 11.

## Claims

1. A lipid emulsion intended for parenteral or enteral nutrition, **characterised in that** the lipid phase, obtained by mixing two or more of the following oils: apricot, almond, peanut, avocado, corn, safflower, rapeseed, cotton, lupin, maize, hazelnut, walnut, olive, evening primrose, palm, peach, grape, rice, rye, sesame; soya, sunflower, tomato, linseed, citrus, is a mixture of long-chain (C > 12) fatty acids, of which 15 to 45 % of the total of fatty acids are essential fatty acids, and **in that** it contains as emulsifiers vegetable, animal or synthetic phospholipids.

2. An emulsion according to claim 1, **characterised in that** it contains a mixture of avocado (46%) and soya (54%) oils.

3. A lipid emulsion according to claim 1, **characterised in that** it contains a mixture of olive (47%) and soya (53%) oils.

4. A lipid emulsion according to claim 1, **characterised in that** it contains a mixture of avocado (60%) and walnut (40%) oils.

5. A lipid emulsion according to claim 1, **characterised in that** it contains a mixture of avocado (89%) and walnut (11%) oils.

6. A lipid emulsion according to claim 1, **characterised in that** it contains a mixture of walnut (11%) and olive (89%) oils.

7. A lipid emulsion according to claim 1, **characterised in that** it contains a mixture of olive (85%) and soya (15%) oils.

8. A lipid emulsion according to claim 1, **characterised in that** it contains a mixture of walnut (40%) and olive (60%) oils.

9. A lipid emulsion according to any one of claims 1 to 8, **characterised in that** it contains from 5 to 50 % by weight of oils in the water.

10. A lipid emulsion according to any one of claims 1 to 9, **characterised in that** it contains as emulsifiers vegetable, animal or synthetic phospholipids, in a proportion of from 0.5 to 5 % by weight of the emulsion.

11. A lipid emulsion according to any one of claims 1 to 10, **characterised in that** it contains a tonicity-regulating agent.

12. An emulsion or solution intended for parenteral or enteral nutrition, **characterised in that** it contains, among its constituents, the lipid emulsion defined in any one of claims 1 to 11.

13. A pharmaceutical composition, **characterised in that** it is constituted by a lipid emulsion as defined in any one of claims 1 to 11.

14. A pharmaceutical composition, **characterised in that** it contains, as constituent, a lipid emulsion as defined in any one of claims 1 to 11.

15. A dietetic composition, **characterised in that** it is constituted by a lipid emulsion as defined in any one of claims 1 to 11.

16. A dietetic composition, **characterised in that** it contains, as constituent, a lipid emulsion as defined in any one of claims 1 to 11.

## Patentansprüche

1. Lipidemulsion zur parenteralen oder enteralen Ernährung, **dadurch gekennzeichnet, dass** die Lipidphase, die aus der Mischung von zwei oder mehreren der nachfolgenden Öle, wie Aprikosenöl, Mandelöl, Erdnussöl, Avocadoöl, Weizenöl, Färberdistelöl, Rapsöl, Baumwollöl, Wolfsbohnenöl, Maisöl, Walnussöl, Olivenöl, Nachtkerzenöl, Palmöl, Fischöl, Traubenöl, Reisöl, Roggenöl, Sesamöl, Sojaöl, Sonnenblumenöl, Tomatenöl, Leinenöl, Zitronenöl erhalten ist, eine Mischung von langkettigen Fettsäuren (C >12) ist, bei der 15% bis 45% der Fettsäuren essentielle Fettsäuren sind, und dass sie als Emulgatoren pflanzliche, tierische oder synthetische Phospholipide enthält.

2. Emulsion nach Anspruch 1, **dadurch gekennzeichnet, dass** sie eine Mischung aus Avocadoöl (46%) und Sojaöl (54%) enthält.

3. Lipidemulsion nach Anspruch 1, **dadurch gekennzeichnet, dass** sie eine Mischung aus Olivenöl (47%) und Sojaöl (53%) enthält.

4. Lipidemulsion nach einem der Ansprüche 1, **dadurch gekennzeichnet, dass** sie eine Mischung aus Avocadoöl (60%) und Nussöl (40%) enthält.

5. Lipidemulsion nach Anspruch 1, **dadurch gekennzeichnet, dass** sie eine Mischung aus Avocadoöl (89%) und Nussöl (11%) enthält.

6. Lipidemulsion nach Anspruch 1, **dadurch gekennzeichnet, dass** sie eine Mischung aus Nussöl (11 %) und Olivenöl (89%) enthält.

7. Lipidemulsion nach Anspruch 1, **dadurch gekennzeichnet, dass** sie eine Mischung aus Olivenöl (85%) und Sojaöl (15%) enthält.

8. Lipidemulsion nach Anspruch 1, **dadurch gekennzeichnet, dass** sie eine Mischung aus Nussöl (40%) und Olivenöl (60%) enthält.

9. Lipidemulsion nach einem der Ansprüche 1 bis 8, **dadurch gekennzeichnet, dass** sie 5 bis 50 Gewichts-% Öl in Wasser enthält.

10. Lipidemulsion nach einem der Ansprüche 1 bis 9, **dadurch gekennzeichnet, dass** sie als Emulgatoren pflanzliche, tierische oder synthetische Phospholipide im Bereich zwischen 0,5 und 5 Gewichts-% der Emulsion enthält.

11. Lipidemulsion nach einem der Ansprüche 1 bis 10, **dadurch gekennzeichnet, dass** sie einen isotonisierenden Wirkstoff enthält.

12. Emulsion oder Lösung zur enteralen oder parenteralen Ernährung, **dadurch gekennzeichnet, dass** sie neben anderen Bestandteilen die Lipidemulsion nach einem der Ansprüche 1 bis 11 enthält.

13. Pharmazeutische Zusammensetzung, **dadurch gekennzeichnet, dass** sie von einer Lipidemulsion gebildet ist, wie sie in einem der Ansprüche 1 bis 11 enthalten ist.

14. Pharmazeutische Zusammensetzung, **dadurch gekennzeichnet, dass** sie als Bestandteil eine Lipidemulsion enthält, wie sie in einem der Ansprüche 1 bis 11 enthalten ist.

15. Diätetische Zusammensetzung, **dadurch gekennzeichnet, dass** sie eine Lipidemulsion enthält, wie sie in einem der Ansprüche 1 bis 11 enthalten ist.

16. Diätetische Zusammensetzung, **dadurch gekennzeichnet, dass** sie als Bestandteil eine Lipidemulsion enthält, wie sie in einem der Ansprüche 1 bis 11 enthalten ist.
